# EUROPEAN PATENT SPECIFICATION

(11) **EP 4 159 175 B1**
(45) Date of publication and mention of the grant of the patent: **15.01.2025**
(21) Application number: 22198992.4
(22) Date of filing: 30.09.2022
(51) Int. Cl.: A61F 13/49, A61F 13/494

(54) **ABSORBENT GARMENT AND METHOD OF MANUFACTURE THEREOF**
ABSORBIERENDES KLEIDUNGSSTÜCK UND VERFAHREN ZU DESSEN HERSTELLUNG
VÊTEMENT ABSORBANT ET SON PROCÉDÉ DE FABRICATION

(30) Priority: 04.10.2021 US 202163251930 P; 28.09.2022 US 202217936060
(43) Date of publication of application: 05.04.2023
(73) Proprietor: Mast Industries (Far East) Limited, Kowloon (HK)
(72) Inventor: Yip, Suet Hing, Tai Wai (HK); Au, Fung Yee Debby, Tai Po (HK); Williams, David John, Sai Kung (HK); Samarasinghe, Meloy Ted, Matara (LK); Wong, Hin Ting Scarlet, Shatin (HK)
(74) Representative: Gill Jennings & Every LLP

(56) References cited:
- US-A1- 2021 177 676

## Description

### FIELD

The present application relates to garments, and more specifically to garments with an absorbent gusset that is configured to be worn adjacent a wearer's crotch.

### BACKGROUND

U.S. Patent Application Publication No. 2014/0039432 discloses an undergarment including a multilayer leak-proof pad to wick moisture, impede bacteria, prevent leaks, and resist stain.

U.S. Patent Application Publication No. 2018/0014983 discloses a liquid absorbing pad for use in clothing, comprising: a liquid permeable layer; a functional layer over the liquid impermeable layer; and a liquid impermeable barrier material, wherein the functional layer is capable of acquiring and distributing liquid and/or absorbing liquid and the liquid impermeable barrier material is bonded to at least the liquid impermeable layer and the functional layer around the periphery of said layers by a bonding means, provided that the bonding means is not stitching. The liquid absorbable pad may form part of a garment.

U.S. Patent Application Publication No. 2020/0222256 discloses a protective insert operatively attachable to an inner, body-facing layer of a garment. The protective insert generally comprises, a first, operatively inner layer comprising a moisture-wicking, odor resistance, fluid absorbent fiber with or without a waterproof laminate-film; and a second, operatively outer layer comprising a breathable, odor resistant, water repellent fiber to further prevent fluid passage through the garment; wherein the operatively inner layer faces the body of a user while the operatively outer layer faces away from the body of a user, in use

U.S. Patent Application Publication No. 2020/0246203 discloses an undergarment article having a fabric layer for wearing about a pelvic region of a person, a water-repellent layer coupled to a crotch portion of the fabric layer to form an inner chamber, and an inner pad disposed within the inner chamber between the water-repellent layer and the fabric layer. The inner pad is resistant to heat and includes a liquid-absorbent layer and a waterproof layer. The liquid-absorbent layer contacts the water-repellent layer and is effective for absorbing a volume of liquid greater than 2 fluid ounces. The waterproof layer is effective for restricting liquid from reaching the fabric layer. The crotch portion of the fabric layer and the water-repellent layer can be substantially elastic in at least one of a lateral direction and a longitudinal direction of the crotch portion. Methods for using the undergarment, kits and uses of the undergarment are also disclosed.

U.S. Patent No. 7,322,966 discloses a washable, reusable garment for retention of body fluids when worn on a person's lower body part. The garment has a crotch portion and a body portion. The crotch portion has an absorbent composite, and a pocket adapted for receiving a removable absorbent pad. The body portion has a waist opening and is seamless except where attached to the crotch portion. The body portion and the crotch portion together form first and second leg openings.

U.S. Patent No. 8,117,675 discloses a waterproof panty that has rolled over welded seams. The rolled over welded seam will inhibit the leaking of bodily fluids at the leg openings caused by stitching and wicking. The panty has an outer shell and an inner panty layer. The outer shell a continuous cut formed of a soft blend laminated fabric. It is liquid proof, breathable, hypoallergenic, stain resistant, and elastic. It is cut to form a waist opening and two leg openings. If desired stretchable lace or elastic side portions can be provided. The inner panty lining is a breathable soft blend fabric and includes a front portion, back portion and a crotch portion. The crotch portion is double layered for added dryness. The inner panty layer is cut to the full design of the panty having a waist opening and two leg openings. This overall design provides the user peace of mind and security with a stylish, lightweight, comfortable, waterproof panty.

U.S. Patent No. 8,460,265 discloses a washable female undergarment designed for comfort and for alleviating leakage. The undergarment comprises an outer layer of a stretchable fabric, an inner layer of an absorbent fabric, and a main absorbent layer positioned between the inner and outer layers in the crotch area. The three layers are permanently secured together to form a reusable reliant undergarment.

U.S. Patent No. 10,231,885 discloses an undergarment configured to help absorb leaks of bodily fluids from a person suffering from mild to moderate incontinence or other conditions including a main body made of a material that allows the absorption of bodily fluids and a crotch piece made of a material that allows the absorption of bodily fluids that overlays the crotch portion of the main body. The crotch piece of the undergarment can extend high enough and be wide enough on the front side of the garment's main body so as to offer an adequate area of protection to the user.

U.S. Patent No. 10,441,479 discloses an undergarment including a body portion having an upper edge defining a waistband, two leg openings and a crotch region between the leg openings; an absorbent pad on the inside of the body portion within at least the crotch region, the absorbent pad having an inner region and a peripheral region, wherein the thickness of the absorbent pad in the peripheral region is less than the thickness of the absorbent pad in the inner region; and elastic bonding film lining each of the two leg openings while overlying respective portions of the peripheral region of the absorbent pad thereby to bond the absorbent pad and the body portion. A method of manufacturing an undergarment is provided.

U.S. Patent 10,555,841 discloses protective garments comprising an inner surface or portion of an inner surface with both absorbent and stain resistant properties while maintaining the soft feel, breathability and aesthetic properties associated with traditional "non protective" intimate apparel. The fabrics and methods of constructing the garments are also disclosed herein.

International Patent Application Publication No. 2019/162615 discloses leaktight underpants comprising a fabric body with a waist opening and a pair of leg openings. They comprise a front part and a rear part, the front and rear parts being fastened together by lateral seams. The leaktight underpants comprise, in the crotch part, a protective assembly, the protective assembly comprising an absorbent layer and a draining layer, which are fastened to the edge portion of each leg opening. The absorbent layer or the draining layer is fastened to the lateral seams of the front or rear parts of the fabric body, only the absorbent layer, or only the draining layer, being fastened to the rear edge or to the front edge, respectively, delimiting the waist opening.

International Patent Application Publication No. 2020/121176 discloses a multi-layer liquid absorbing and retaining textile assembly. The textile assembly comprises at least a first layer, a second layer and an intermediate layer. The intermediate layer is constructed as a warp knitted spacer fabric having a plurality of sections with at least one section being proximal to the first layer and at least another section being proximal to the second layer. The first layer is adapted to transfer liquid from a first side of the textile assembly to the intermediate layer. The intermediate layer is adapted to receive and transfer liquid to the second layer. The second layer is adapted to retain the liquid at or proximal to a second side of the textile assembly that is oppositely facing to the first side.

U.S. Patent No. 11,154,431 discloses a garment comprising a main body including a front portion, a back portion, and a gusset connecting the front portion and the back portion. The gusset has left and right lateral edges partially defining respective left and right leg openings of the garment. A layer of absorbent material is coupled to an inner face of the main body at least at the gusset. A layer of waterproof material is sandwiched between the main body and the layer of absorbent material. Left and right elastic bands couple the layer of absorbent material and the layer of waterproof material to the main body along the respective left and right lateral edges of the gusset. A method for manufacturing the absorbent garment is also provided.

U.S. Patent Application Publication No. 2021/0177676 discloses an absorbent pad for use in a garment. The absorbent pad includes: a liquid impermeable barrier layer; a functional layer attached to the barrier layer, the functional layer comprising a liquid absorbent component; a liquid impermeable peripheral sealing element bonded to peripheries of the functional layer and barrier layer; and a peripheral attaching element having a first portion and a second portion, the first portion bonded to the peripheral sealing element. The second portion of the peripheral attaching element is detached from the peripheral sealing element and is arranged to be attached to a fabric body of the garment, thereby attaching the absorbent pad to the garment.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

According to one example, a garment comprises a main body including a front portion, a back portion, and a gusset connecting the front portion and the back portion. The gusset has left and right lateral edges partially defining respective left and right leg openings of the main body. An absorbent material is coupled to an inner face of the main body at least at the gusset. A waterproof material is sandwiched between the main body and the absorbent material. Left and right elastic bands are coupled to outer lateral edges of the absorbent material. The left and right elastic bands are bonded to the waterproof material and the waterproof material is attached to the main body proximate the respective left and right lateral edges of the gusset. The left and right elastic bands are folded around respective left and right lateral edges of the absorbent material. The waterproof material is attached to the main body between respective bottom halves of the folds of each of the left and right elastic bands and the inner face of the main body.

Optionally, the waterproof material is bonded to each of the left and right elastic bands at laterally outer edges of the waterproof material using bonding film, and the waterproof material extends outwardly beyond the bonding film to provide free edges that are attached to the main body.

Optionally, the left and right elastic bands are bonded to the absorbent material where the left and right elastic bands overlap the respective left and right lateral edges of the absorbent material.

In one example, a liner material is coupled to the absorbent material such that the absorbent material is sandwiched between the liner material and the waterproof material. Optionally, the left and right elastic bands are folded around respective left and right lateral edges of each of the liner material and the absorbent material and the left and right elastic bands are bonded to the liner material and the absorbent material where the left and right elastic bands overlap the respective left and right lateral edges of each of the liner material and the absorbent material.

In one example, left and right lateral edges of a material of the main body are folded over toward the inner face of the main body at the gusset to define the left and right lateral edges of the gusset, and left and right lateral edges of the waterproof material are attached to the respective left and right lateral edges of the main body material. Optionally, the left and right lateral edges of the waterproof material are attached to the respective left and right lateral edges of the main body material by stitching. Optionally, the left and right elastic bands are folded around respective left and right lateral edges of the absorbent material and the left and right elastic bands respectively overlap the stitching between the left and right lateral edges of the waterproof material and the respective left and right lateral edges of the main body material. Optionally, the main body material is bonded to itself where the left and right lateral edges of the main body material are folded over, and the main body is substantially seamless on an outer face thereof at least at the gusset.

In one example, the waterproof material comprises a fabric bonded to a waterproof membrane.

According to another example of the present disclosure, a method for manufacturing an absorbent garment comprises providing a main body including a front portion, a back portion, and a gusset connecting the front portion and the back portion, wherein the gusset has left and right lateral edges configured to partially define respective left and right leg openings of the main body; providing an absorbent material sized and shaped to fit on the gusset and having left and right lateral edges configured to extend alongside the respective left and right lateral edges of the gusset; attaching left and right elastic bands along the respective left and right lateral edges of the absorbent material; providing a waterproof material sized and shaped to fit on the gusset and having left and right lateral edges configured to extend alongside the respective left and right lateral edges of the gusset; attaching the left and right lateral edges of the waterproof material to the main body proximate the respective left and right lateral edges of the gusset such that the waterproof material is located on an inner face of the main body at the gusset; and placing the attached absorbent material and left and right elastic bands on the gusset over the waterproof material and attaching the left and right elastic bands to the waterproof material such that the waterproof material is sandwiched between the absorbent material and the main body.

In one example, the method further comprises folding the left and right elastic bands around the respective left and right lateral edges of the absorbent material. Optionally, the method further comprises bonding the left and right elastic bands to the absorbent material where the left and right elastic bands overlap the respective left and right lateral edges of the absorbent material.

In one example, the method further comprises folding left and right lateral edges of a material of the main body over toward an inner face of the main body at the gusset to define the left and right lateral edges of the gusset, and attaching the left and right lateral edges of the waterproof material to the respective left and right lateral edges of the main body material. Optionally, the attaching of the left and right lateral edges of the waterproof material to the respective left and right lateral edges of the main body material is done by stitching. Optionally, the left and right elastic bands respectively overlap the stitching between the left and right lateral edges of the waterproof material and the respective left and right lateral edges of the main body material. Optionally, the method further comprises bonding the main body material to itself where the left and right lateral edges of the main body material are folded over such that the main body is substantially seamless on an outer face thereof at least at the gusset.

In one example, the attaching of the left and right elastic bands along the respective left and right lateral edges of the absorbent material is done by bonding.

In one example, the attaching of the left and right lateral edges of the waterproof material to the main body proximate the respective left and right lateral edges of the gusset is done by stitching.

In one example, the method further comprises providing a liner material sized and shaped to fit on the gusset and having left and right lateral edges configured to extend alongside the respective left and right lateral edges of the gusset; attaching the left and right elastic bands along the respective left and right lateral edges of each of the absorbent material and the liner material; and placing the attached liner material, absorbent material, and left and right elastic bands on the gusset over the waterproof material and attaching the left and right elastic bands to the waterproof material such that the absorbent material is sandwiched between the liner material and the waterproof material.

### BRIEF DESCRIPTION OF THE DRAWINGS

Examples of garments and portions thereof are described with reference to the following Figures. The same numbers are used throughout the Figures to reference like features and like components.
FIGURE 1 is a front view of a garment according to the present disclosure.
FIGURE 2 is an interior view of the gusset of the garment.
FIGURE 3 is a schematic cross-sectional view taken through the gusset of the panty along the line 3-3 shown in FIGURE 2.
FIGURE 4 is a partial cut-away view of the gusset of the panty.
FIGURE 5 illustrates a method of manufacturing the panty according to the present disclosure.

### DETAILED DESCRIPTION

The present application relates to garments, and more specifically to garments having an absorbent and waterproof gusset area configured to cover a wearer's crotch, although the absorbent and waterproof portions of the garment could extend beyond the gusset area as well. For example, the present application relates to panties or swimwear, or to garments such as shorts, pants, or skirts having an integral panty, capable of absorbing bodily fluids such as urine, menstrual fluid, vaginal discharges, and/or sweat without allowing the bodily fluid to seep through to an outer face of the garment. The panties, swimwear, or garment having an integral panty can be any style and can have any trim, which details are not limiting on the scope of the present disclosure. In other examples, the garment is a pair of tights, a pair of pantyhose, a bodysuit, or any other type of garment that is configured to be worn directly against a wearer's skin.

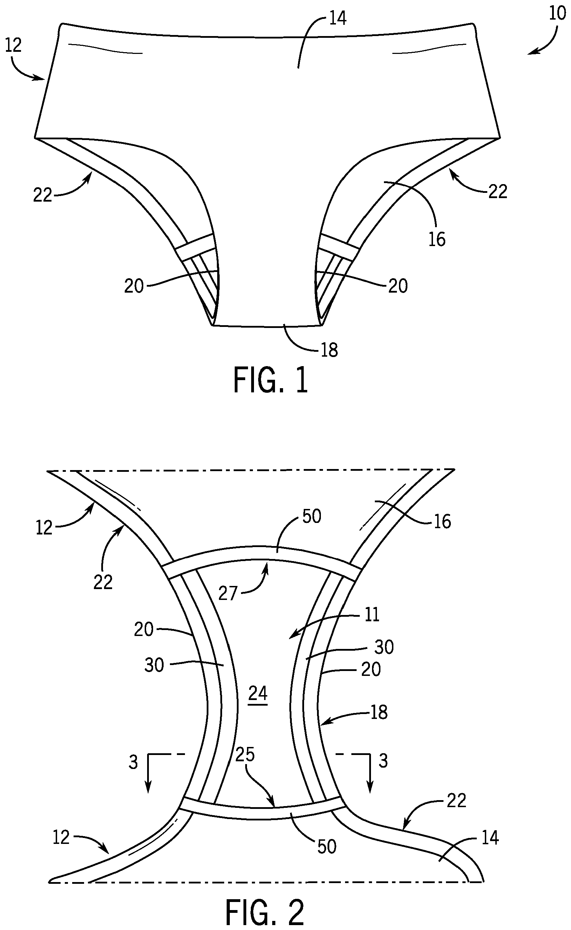

FIGURE 1 shows a front view of one example of a garment 10. Here, the garment 10 is a pair of panties, although note the garment 10 could instead be a pair of swim bottoms. The garment 10 has a main body 12 including a front portion 14, a back portion 16, and a gusset 18 connecting the front portion 14 and the back portion 16. The gusset 18 has opposite left and right lateral edges 20 partially defining respective left and right leg openings 22 of the main body 12.

FIGURE 2 shows the interior of the garment 10, with a portion of the gusset 18 shown in detail. A liner material 24 can be seen covering a majority of the gusset 18. The liner material 24 shows the extent of an absorbent assembly 11 of the garment 10 and covers an absorbent material and a waterproof material, which are also coupled to the gusset 18 as will be described further herein below. While the absorbent assembly 11 is coupled to the garment 10 at least at the gusset 18, it could extend into the front portion 14 and/or back portion 16, as noted hereinabove. Options for the absorbent assembly 11 of the garment 10 are described below.

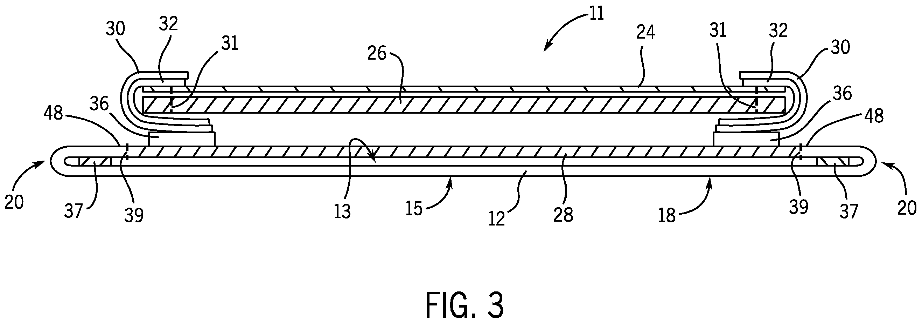

In one example, as shown in FIGURE 3, an absorbent material 26 is coupled to an inner face 13 of the main body 12 of the garment 10 at least at the gusset 18. A waterproof material 28 is sandwiched between the main body 12 and the absorbent material 26. The liner material 24 is coupled to the absorbent material 26 opposite the waterproof material 28, such that the absorbent material 26 is sandwiched between the liner material 24 and the waterproof material 28. In use, the liner material 24 is configured to touch the wearer's skin while the garment 10 is worn. However, as will be described herein below, the liner material 24 is optional. Left and right elastic bands 30 are coupled to the outer lateral edges of the optional liner material 24, the absorbent material 26, and the waterproof material 28, as will be described in further detail below. The waterproof material 28 is attached to the main body 12 proximate the respective left and right lateral edges 20 of the gusset 18.

In one example, the left and right elastic bands 30 are folded around respective left and right lateral edges of each of the liner material 24 and the absorbent material 26 to form a "V" shape and then attached to the layers of liner material 24 and absorbent material 26 along the opposite lateral edges thereof. For example, the left and right elastic bands 30 are bonded to the liner material 24 and the absorbent material 26 where the left and right elastic bands 30 overlap the respective left and right lateral edges of each of the liner material 24 and the absorbent material 26, such as by way of bonding film 32. In some examples, the bonding film 32 lines the entire "V" of each folded elastic band 30, but in other examples separated strips of bonding film are used on the top and bottom folds of the "V." In other examples, the bonding is done by way of spray glue or other adhesive application. Before the elastic bands 30 are attached, the liner material 24 can optionally be bonded to the absorbent material 26 by way of heat-activated adhesive tape and/or can be stitched to the absorbent material 26 as shown at stitching 31. Stitching 31 can be intermittent along the length of the gusset 18, and meant simply to hold the liner material 24 and absorbent material 26 in place with respect to each other while the elastic bands 30 are attached thereto. If the absorbent material 26 and the liner material 24 are stitched together before the elastic bands 30 are attached thereto, the absorbent material 26 can optionally also be stitched to the bottom half of the fold of each of the elastic bands 30. The top half of the fold of each elastic band 30 could then be folded over and bonded to the top of the liner material 24.

In one example, before the attached absorbent material 26, liner material 24, and elastic bands 30 are assembled to the main body 12, the outer lateral edges of the waterproof material 28 are attached to the fabric of the main body 12. More specifically, left and right lateral edges 48 of the material of the main body 12 are folded over toward the inner face 13 of the main body 12 at the gusset 18 to define the left and right lateral edges 20 of the gusset 18. Left and right lateral edges of the waterproof material 28 are attached to the respective left and right lateral edges 48 of the main body 12 material. In one example, the left and right lateral edges of the waterproof material 28 are attached to the respective left and right lateral edges of the main body 12 material by stitching, as shown at 39, which can be pullout overlock, cover stitch, or other known stitching. In other examples, the waterproof material 28 is bonded to the main body 12 material at the lateral edges of each material. Optionally, the main body 12 material is bonded to itself where the left and right lateral edges 48 of the main body 12 material are folded over using bonding film 37. Thus, the main body 12 is substantially seamless on an outer face 15 thereof at least at the gusset 18. However, the stitching 39 attaching the waterproof material 28 to the material of the main body 12 provide integrity to the garment 10 that is not present in prior art garments that are only bonded.

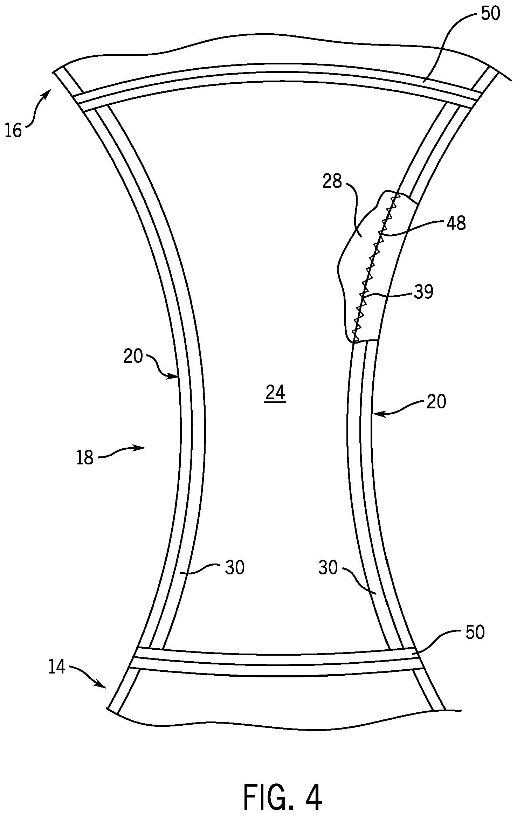

After the liner material 24 and absorbent material 26 are bonded and/or stitched to each other and to the elastic bands 30, the left and right elastic bands 30 are bonded to the waterproof material 28, which has optionally been pre-attached to the main body 12. More specifically, the waterproof material 28 can be bonded to each of the left and right elastic bands 30 at laterally outer edges of the waterproof material 28 using bonding film 36. If the liner material 24 and the absorbent material 26 are stitched to the bottom half of the fold of each elastic band 30, the bonding film 36 can be placed over stitches by which the left and right elastic bands 30 are stitched to the respective left and right lateral edges of each of the absorbent material 26 and the liner material 24. The liner material 24, absorbent material 26, and elastic bands 30 are sized and shaped such that when they are placed on the gusset 18, the left and right elastic bands 30 respectively overlap the stitching 39 between the left and right lateral edges of the waterproof material 28 and the respective left and right lateral edges 48 of the main body 12 material to hide the stitching 39 from view and protect it from contact. This is also shown in the partial cutaway view provided in FIGURE 4, in which the folded-over edge 48 of the material of the main body 12 is shown as being stitched to the outer lateral edge of the waterproof material 28 at stitching 39, which is covered by elastic band 30. Optionally, the elastic bands 30 can also be bonded to the stitching 39 and/or to the folded-over lateral edges 48 of the main body 12, such as if the bonding film 36 is extended outwardly on both sides of the gusset 18 to cover over the stitching 39 and extend to or over the main body 12 fabric.

Because the left and right elastic bands 30 are bonded and/or stitched to the absorbent material 26 and the liner material 24 and bonded to the waterproof material 28, this lessens the likelihood of leaks. The bonding film 32 effectively creates a "cuff' around the absorbent portion(s) 24, 26 of the absorbent assembly 11, which cuff together with the waterproof material 28 prevents bodily fluids from seeping to the fabric of the main body 12. The elastic bands 30 may be treated with chemicals that make them waterproof or water resistant, as described herein below, which can further prevent leaks from the absorbent portion(s) of the absorbent assembly 11. Furthermore, because the waterproof material 28 is bonded to each of the left and right elastic bands 30 over any stitches by which the left and right elastic bands 30 are optionally stitched to each of the respective left and right lateral edges of the absorbent material 26, any liquid that might otherwise tend to leak from the absorbent portion(s) 24, 26 of the absorbent assembly 11 due to such stitching 31 is maintained on the inner face of the waterproof material 28 (i.e., not on the face that contacts the main body 12 fabric).

The elastic bands 30 can optionally be asymmetrically folded, as shown in FIGURE 3, such that more of the elastic band 30 is on the bottom part of the fold than on the top part of the fold. This provides more surface area for the waterproof material 28 to be bonded to the bottom part of the folded elastic band 30 at bonding film 36, while providing less surface area that contacts the wearer's skin on the upper surface of the absorbent assembly 11. The waterproof material 28 extends outwardly beyond the bonding film 36 to provide the free edges that are be attached to the material of the main body 12. As will be described herein below, the waterproof material 28 can include a fabric bonded to a waterproof membrane, and either the fabric side or the membrane side can be the side that is bonded to the elastic bands 30 via the bonding film 36. In a preferred example, the fabric side of the waterproof material 28 is bonded to the elastic bands 30 via the bonding film 36. This is because the fabric side of the waterproof material 28 has some ability to absorb moisture, thereby attracting any moisture that leaks from the elastic bands 30 and/or from the absorbent material 26. Such leaks are then eventually stopped by the waterproof membrane side of the waterproof material 28, which faces the inner face 13 of the main body 12.

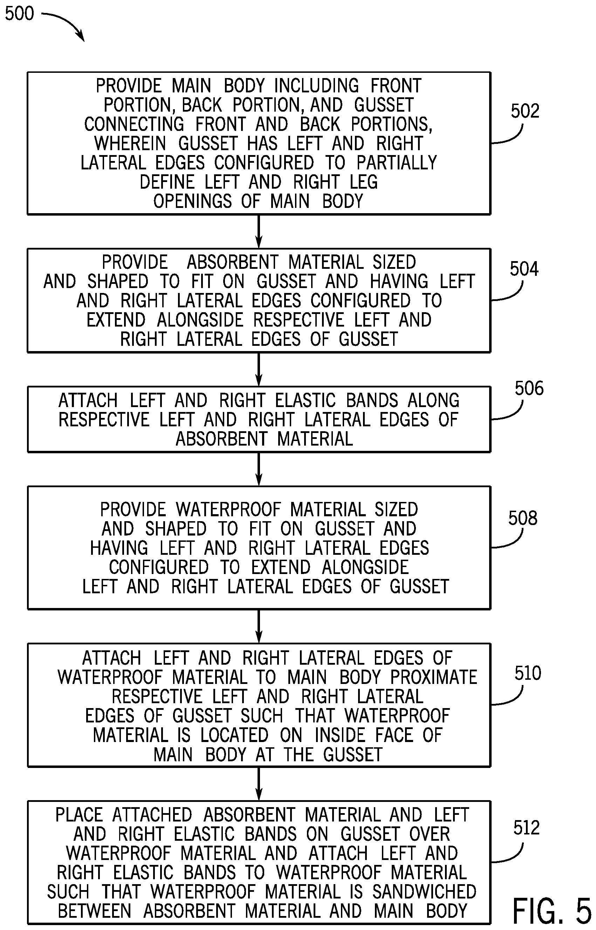

As shown in FIGURE 5, a method 500 for manufacturing an absorbent garment 10 is also disclosed. The method comprises providing a main body 12 including a front portion 14, a back portion 16, and a gusset 18 connecting the front portion 14 and the back portion 16, wherein the gusset 18 has opposite left and right lateral edges 20 configured to partially define respective left and right leg openings 22 of the main body 12, as shown at 502. As shown at 504, the method includes providing an absorbent material 26 sized and shaped to fit on the gusset 18 and having left and right lateral edges configured to extend alongside the respective left and right lateral edges 20 of the gusset 18. Note that the absorbent material 26 can have the exact same (or similar) shape as the gusset 18, but is slightly smaller in the lateral direction than the gusset 18, such that the absorbent material 26 can fit on the gusset 18 without hanging over the lateral edges 20 thereof.

The method includes attaching left and right elastic bands 30 along the respective left and right lateral edges of the absorbent material 26, as shown at 506. For example, as shown and described above, the method includes folding the left and right elastic bands 30 around the respective left and right lateral edges of the absorbent material 26. The method may further include bonding the left and right elastic bands 30 to the absorbent material 26 where the left and right elastic bands 30 overlap the respective left and right lateral edges of the absorbent material 26, as shown at bonding film 32. Note that in this example of the method, no liner material 24 is provided. This may be desirable if the finished garment 10 is to be lightweight and thin in the gusset 18. For example, the absorbent material 26 could be the layer that touches the wearer's skin (and thus acts as a dual "liner"/absorbent layer). As will be described further herein below, this liner/absorbent material 26 can be a push-pull fabric. In other examples, however, the method may include providing a liner material 24 sized and shaped to fit on the gusset 18 and having left and right lateral edges configured to extend alongside the respective left and right lateral edges 20 of the gusset 18. Such a method may include attaching the left and right elastic bands 30 along the respective left and right lateral edges of each of the absorbent material 26 and the liner material 24, for example by bonding.

Continuing with FIGURE 5, as shown at 508, the method includes providing a waterproof material 28 sized and shaped to fit on the gusset 18 and having left and right lateral edges configured to extend alongside the respective left and right lateral edges 20 of the gusset 18. Note that the waterproof material 28 can have the exact same (or similar) shape as the gusset 18, but is slightly smaller in the lateral direction than the gusset 18. This way, the waterproof material 28 can fit on the gusset 18 without hanging over the lateral edges 20 thereof, but is still wide enough to attach to the folded over lateral edges 48 of the main body 12 material. To that end, the method also includes attaching the left and right lateral edges of the waterproof material 28 to the main body 12 proximate the respective left and right lateral edges 20 of the gusset 18 such that the waterproof material 28 is located on an inner face 13 of the main body 12 at the gusset 18, as shown at 510. For example, the attaching of the left and right lateral edges of the waterproof material 28 to the respective left and right lateral edges 48 of the main body 12 material may be done by stitching. Optionally, the method includes bonding the main body 12 material to itself where the left and right lateral edges 48 of the main body 12 material are folded over such that the main body 12 is substantially seamless on an outer face thereof 15 at least at the gusset 18. In one particular, non-limiting example, the waterproof material 28 may be stitched to the edges 48 of the main body 12 fabric in a bagged-out configuration, after which the bonding film 37 can be applied to the edges of the main body 12 adjacent the stitching 39, and the main body 12 can be turned right-side-out. When the main body 12 is turned right-side-out and heat-treated along the edges 48 thereof, the edges 48 of the main body 12 are bonded together by the bonding film 37 where they are folded over. Other methods for attaching the waterproof material 28 to the main body 12 may be used.

As shown at 512, the method includes placing the attached absorbent material 26 and left and right elastic bands 30 on the gusset 18 over the waterproof material 28 and attaching the left and right elastic bands 30 to the waterproof material 28 such that the waterproof material 28 is sandwiched between the absorbent material 26 and the main body 12. As noted hereinabove, this portion of the method may include folding left and right lateral edges 48 of the material of the main body 12 over toward the inner face 13 of the main body 12 at the gusset 18 to define the left and right lateral edges 20 of the gusset 18, and attaching the left and right lateral edges of the waterproof material 28 to the respective left and right lateral edges 48 of the main body 12 material. Optionally, as shown in FIGURES 3 and 4, the left and right elastic bands 30 may respectively overlap the stitching 39 between the left and right lateral edges of the waterproof material 28 and the respective left and right lateral edges 48 of the main body 12 material.

In examples in which a liner material 24 is present, the method may include placing the attached liner material 24, absorbent material 26, and left and right elastic bands 30 on the gusset 18 over the waterproof material 28 and attaching the left and right elastic bands 30 to the waterproof material 28 such that the absorbent material 26 is sandwiched between the liner material 24 and the waterproof material 28.

In other examples, the elastic bands 30, absorbent material 26, and optional liner material 24 are attached to the waterproof material 28 before the waterproof material 28 is attached to the material of the main body 12. In other examples, instead of having folded-over edges 48, the edges of the main body 12 are raw cut.

Any of the above stitched connections could instead be made by bonding, and vice versa, although the present inventors have found that the connections specifically shown and described herein are suitable for strength and waterproofing purposes. For instance, the waterproof material 28 is sewn to the material of the main body 12 to provide a strong, washable connection that lasts longer than prior art bonded connections along the leg openings 22. The bonded connections between the elastic bands 30 and the waterproof material 28 ensure that liquid absorbed by the absorbent material 26 does not leak out of the absorbent portion(s) of the garment 10, as might otherwise occur with a stitched connection between these pieces. The elastic bands 30 can be treated with a water-repellant or waterproof finish that makes them resistant to water intrusion, thereby further ensuring that liquid stays inside the absorbent portion(s) of the garment 10. Any thread used for stitching, especially at stitching 39, can also be treated with a water-repellant or waterproof finish.

The liner material 24 can be any appropriate material that quickly absorbs liquid and pulls it away from the body. In one example, the liner material 24 is a jersey knit material made of, for example, cotton or a cotton-synthetic blend. In one particular example, the liner material 24 is a 91% cotton (40S), 9% elastane (40D) weft knit single jersey fabric. In another example, the liner material 24 is a French terry knit. The liner material 24 may be treated to provide special functionality, such as with a stain-release finish. In one example, the liner material 24 is manufactured from polyester stain-release yarn, such as TOP CLEAN^{™} from Far Eastern New Century Corporation of Taiwan. For example, if the fabric is French terry knit, the terry side can be made of stain-release polyester yarn and the flat side can be made of regular polyester yarn. In another example, the liner material 24 is treated on the non-skin side with a chemical that causes the fabric to pull liquid away from its skin-facing surface, and thereafter prevents the liquid from returning to the skin-facing side of the fabric. In still another example, a push-pull effect can be provided by yarn choice and fabric construction, such as if the skin-facing side of the liner material 24 is constructed of yarn which is liquid-repellant, and the non-skin side is constructed of yarn that is liquid-absorbent. The liquid will be pushed by the liquid-repellant side to go through to the water-absorbent side, which simultaneously pulls the water away from the skin-facing, liquid-repellant side of the fabric. In still another example, the liner material 24 is treated with an anti-microbial finish. In one particular example, the liner material 24 can be treated with AGION^{™} AM Slurry available from Sciessent LLC of Wakefield, Massachusetts. In another particular example, the liner material 24 can be treated with Sanitized^{™} T20-19 anti-microbial finish from Sanitized AG of Switzerland and/or HeiQ Fresh FFL odor control finish from HeiQ AG of Switzerland. Any of the above-noted functionalities and/or treatments of the liner material 24 can be combined with one another.

The absorbent material 26 can be any appropriate material that is capable of absorbing a volume of liquid up to, for example, 20-50 mL when provided in a size corresponding to a typical panty gusset. The absorbent material 26 can be cotton, polyester, or blends thereof. Typically, the fabric will have a medium to heavy weight, ranging from 250gsm-400gsm. The absorbent material 26 can be French terry fabric, with the non-flat side being maintained as longer loops of yarn or being shredded to create fleece. In one example, the absorbent material 26 is a cotton/polyester French terry or fleece fabric with a water-absorbent finish, with the flat side facing the liner material 24 and the non-flat (e.g., fleece) side facing the waterproof material 28. In another example, the absorbent material 26 is a two-layer bonded terry or fleece fabric, the flat side of which has a water-absorbent finish facing the liner material 24 and the non-flat (e.g., fleece) side of which has a water absorbent finish facing the waterproof material 28. In one particular example, the absorbent material 26 is weft-knit French terry, the non-flat side of which has been shredded to form fleece, made of 84% cotton (30S), 16% polyester (10S) having a weight of 340gsm, such as CO3F0024-BR01AV available from Far Eastern Polytex (VN) Ltd. of Binh Duong, Vietnam. In some examples, the absorbent material 26 can be treated with a chemical, knit, and/or by way of yarn choice be constructed to provide a push-pull effect as was described herein above with respect to the liner material 24. This may be particularly advantageous if no separate liner material 24 is provided and the absorbent material 26 directly touches the wearer's skin. In one particular example, the dual-purpose liner/absorbent push-pull fabric is knit specifically to have a surface contact angle against the skin that tends to pull moisture away from the skin in the z-direction by capillary action. The side of the fabric that is configured to be located further from the skin may then spread the moisture throughout and along the far side of the fabric in the x-and y-directions, again by capillary action, such that the moisture is located on the far side of the fabric instead of the side that contacts the skin. In this regard, the dual-purpose liner/absorbent push-pull fabric as a whole is considered to absorb water by capillary action, even if it is knit from yarns of a hydrophobic polymer, such as, for example, polyester.

The waterproof material 28 can be any suitable material that does not allow liquid to pass therethrough or at least is resistant to liquid. In one example, the waterproof material 28 comprises a fabric bonded to a waterproof membrane. The fabric can be a sheer or semi-sheer fabric knitted from nylon, spandex, polyester, or blends thereof. The waterproof membrane can be adhered, laminated, coated, or otherwise integrally formed on or bonded to the fabric. The waterproof membrane can be a polyurethane membrane or a thermoplastic polyurethane (TPU) membrane, which in one example comprises coffee oil to provide odor control. In one particular example, the waterproof material 28 is weft-knit 100% polyester coated with a polyurethane membrane, and the membrane includes coffee oil. One such fabric is KB445S from Singtex Industrial Co., Ltd. of New Taipei City, Taiwan, which is 100% weft-knit polyester coated with a polyurethane membrane that comprises SCAFE^{™} Polyol. Alternatively, the waterproof material 28 can be SCAFE^{™} AIRMEM^{™} from Singtex Industrial Co., Ltd. In another example, the waterproof material 28 is fabric treated with a water repellent finish, which is bonded to a waterproof membrane. In another example, the waterproof material 28 is a fabric treated with a water repellent finish, which is not bonded to a waterproof membrane, for example, a non-woven material coated with a waterproofing chemical.

The main body 12 material can be any fabric suitable for maintaining a close-to-body feel on the wearer's lower torso. For example, the main body 12 fabric can be a cotton-synthetic blend (e.g., cotton-spandex), a synthetic blend (of nylon, elastane, polyester, etc.), or any other known suitable fabric. In one particular example, the main body 12 fabric is a weft knit interlock nylon-elastane blend. In another particular example, the main body 12 fabric is a warp knit tricot nylon-elastane blend. The main body 12 fabric of the gusset 18 can be treated with or made from an odor-controlling material. For example, the fabric of the gusset 18 can be knitted partially or wholly with coffee-containing yarn, such as MYLITHE^{™} yarn from SCAFE^{™}. Alternatively, the main body 12 fabric can be treated with an odor-controlling substance, such as HeiQ Fresh FFL odor control finish, from HeiQ AG of Switzerland. Additionally or alternatively, the main body 12 fabric can be treated with a wicking finish that wicks moisture from the wearer's body to the outer face of the garment 10. Regardless of the fabric used for the gusset 18, any or all of the layers of material 24, 26, 28 can be treated with an antimicrobial finish as described herein above with respect to the liner material 24 and/or the threads of the fabric can include an antimicrobial fiber, such as silver, to neutralize odors.

Referring back to FIGURE 2, the front and back ends 25, 27 of the liner material 24 (or the absorbent material 26 if liner material is not provided) can be bonded to the fabric of the gusset 18 and/or front portion 14 or back portion 16 of the main body 12 by way of heat-activated adhesive finishing tape 50. Alternatively or additionally, the front and back ends of the liner material 24 (or the absorbent material 26) can be sewn to the main body 12. The waterproof material 28 can be bonded on its outer-facing side to the main body 12 at these locations as well. At these locations, the waterproof material 28 can be bonded on its inner-facing side to the absorbent material 26, which in turn can be bonded to the liner material 24 (if present).

Any bonding noted hereinabove can be done by way of elastomeric heat-activated adhesive tape/film, such as that available from BEMIS^{™}. Alternative methods of bonding include using spray adhesive, printed adhesive, mesh adhesive tape, ultrasonic bonding, and/or liquid glue.

The elastic bands 30 noted herein above can be braided, knitted, or woven elastic bands typically available for elasticizing the leg openings and/or waistband of garments.

As noted hereinabove, the garment can be a pair of panties or a swimsuit bottom. Alternatively, the garment can be a pair of shorts, a pair of pants, a skirt, a dress, or the like into which a pair of panties as shown in FIGURE 1 is sewn. The main body 12 in such instances can be stitched or bonded about the waistband thereof into the waistband of the shorts, pants, skirt, etc. A benefit of the seamless outer face 15 of the main body 12 is that when included as a built-in panty in tighter fitting garment, the main body 12 is not visible or at least has less visible seam lines than might otherwise be apparent if there was stitching on the outer face 15 of the main body 12.

In the above description, unless otherwise noted, "inner" refers to a face of a layer that faces the wearer when the garment is worn 10, while "outer" refers to a face of a layer that faces away from the wearer while the garment 10 is worn. The terms "left" and "right" and "front" and "back" are for reference purposes and are not limiting on the scope of the present disclosure or claims.

In the present description, certain terms have been used for brevity, clarity, and understanding. No unnecessary limitations are to be implied therefrom beyond the requirement of the prior art because such terms are used for descriptive purposes only and are intended to be broadly construed. The different assemblies described herein may be used alone or in combination with other systems. Various equivalents, alternatives, and modifications are possible within the scope of the appended claims. The methods herein are not limited to being performed in the order described, but could be performed in any logical order.

## Claims

1. A garment (10) comprising:
a main body (12) including a front portion (14), a back portion (16), and a gusset (18) connecting the front portion (14) and the back portion (16), wherein the gusset (18) has left and right lateral edges (20) partially defining respective left and right leg openings (22) of the main body (12);
an absorbent material (26) coupled to an inner face (13) of the main body (12) at least at the gusset (18);
a waterproof material (28) sandwiched between the main body (12) and the absorbent material (26); and
left and right elastic bands (30) coupled to outer lateral edges of the absorbent material (26);
wherein the left and right elastic bands (30) are bonded to the waterproof material (28); and
wherein the waterproof material (28) is attached to the main body (12) proximate the respective left and right lateral edges (20) of the gusset (18);
**characterized in that**
the left and right elastic bands (30) are folded around respective left and right lateral edges of the absorbent material (26); and
the waterproof material (28) is attached to the main body (12) between respective bottom halves of the folds of each of the left and right elastic bands (30) and the inner face (13) of the main body (12).

2. The garment of claim 1, wherein the waterproof material (28) is bonded to each of the left and right elastic bands (30) at laterally outer edges of the waterproof material (28) using bonding film (36), and the waterproof material (28) extends outwardly beyond the bonding film (36) to provide free edges that are attached to the main body (12).

3. The garment of claim 1, wherein the left and right elastic bands (30) are bonded to the absorbent material (26) where the left and right elastic bands (30) overlap the respective left and right lateral edges of the absorbent material (26).

4. The garment of any of the preceding claims, further comprising a liner material (24) coupled to the absorbent material (26) such that the absorbent material (26) is sandwiched between the liner material (24) and the waterproof material (28).

5. The garment of claim 4, wherein the left and right elastic bands (30) are folded around respective left and right lateral edges of each of the liner material (24) and the absorbent material (26); and
wherein the left and right elastic bands (30) are bonded to the liner material (24) and the absorbent material (26) where the left and right elastic bands (30) overlap the respective left and right lateral edges of each of the liner material (24) and the absorbent material (26).

6. The garment of any of the preceding claims, wherein left and right lateral edges (48) of a material of the main body (12) are folded over toward the inner face (13) of the main body (12) at the gusset (18) to define the left and right lateral edges (20) of the gusset (18); and
wherein left and right lateral edges of the waterproof material (28) are attached to the respective left and right lateral edges (48) of the main body (12) material.

7. The garment of claim 6, wherein the left and right lateral edges of the waterproof material (28) are attached to the respective left and right lateral edges (48) of the main body (12) material by stitching (39).

8. The garment of claim 7,
wherein the left and right elastic bands (30) respectively overlap the stitching (39) between the left and right lateral edges of the waterproof material (28) and the respective left and right lateral edges (48) of the main body (12) material.

9. The garment of any of claims 6 to 8, wherein the main body (12) material is bonded to itself where the left and right lateral edges (48) of the main body (12) material are folded over, and the main body (12) is substantially seamless on an outer face (15) thereof at least at the gusset (18).

10. A method for manufacturing an absorbent garment (10) according to any one of the above claims, the method comprising:
providing the main body (12);
providing the absorbent material (26), which is sized and shaped to fit on the gusset (18) and has left and right lateral edges configured to extend alongside the respective left and right lateral edges (20) of the gusset (18);
attaching the left and right elastic bands (30) along the respective left and right lateral edges of the absorbent material (26);
providing the waterproof material (28), which is sized and shaped to fit on the gusset (18) and has left and right lateral edges configured to extend alongside the respective left and right lateral edges (20) of the gusset (18);
attaching the left and right lateral edges of the waterproof material (28) to the main body (12) proximate the respective left and right lateral edges (20) of the gusset (18) such that the waterproof material (28) is located on the inner face (13) of the main body (12) at the gusset (18); and
placing the attached absorbent material (26) and left and right elastic bands (30) on the gusset (18) over the waterproof material (28) and attaching the left and right elastic bands (30) to the waterproof material (28) such that the waterproof material (28) is sandwiched between the absorbent material (26) and the main body (12).

11. The method of claim 10, further comprising folding left and right lateral edges (48) of a material of the main body (12) over toward the inner face (13) of the main body (12) at the gusset (18) to define the left and right lateral edges (20) of the gusset (18); and
attaching the left and right lateral edges of the waterproof material (28) to the respective left and right lateral edges (48) of the main body (12) material.

12. The method of claim 11, wherein the attaching of the left and right lateral edges of the waterproof material (28) to the respective left and right lateral edges (48) of the main body (12) material is done by stitching.

13. The method of claim 12, wherein the left and right elastic bands (30) respectively overlap the stitching (39) between the left and right lateral edges of the waterproof material (28) and the respective left and right lateral edges (48) of the main body (12) material.

14. The method of any of claims 11 to 13, further comprising bonding the main body (12) material to itself where the left and right lateral edges (48) of the main body (12) material are folded over such that the main body (12) is substantially seamless on an outer face (15) thereof at least at the gusset (18).

15. The method of any of claims 10 to 14, further comprising:
providing a liner material (24) sized and shaped to fit on the gusset (18) and having left and right lateral edges configured to extend alongside the respective left and right lateral edges (20) of the gusset (18);
attaching the left and right elastic bands (30) along the respective left and right lateral edges of each of the absorbent material (26) and the liner material (24); and
placing the attached liner material (24), absorbent material (26), and left and right elastic bands (30) on the gusset (18) over the waterproof material (28) and attaching the left and right elastic bands (30) to the waterproof material (28) such that the absorbent material (26) is sandwiched between the liner material (24) and the waterproof material (28).

## Patentansprüche

1. Kleidungsstück (10), umfassend:
einen Hauptkörper (12), welcher einen Vorderabschnitt (14), einen Hinterabschnitt (16) und einen Zwickel (18) beinhaltet, welcher den Vorderabschnitt (14) und den Hinterabschnitt (16) verbindet, wobei der Zwickel (18) eine linke und eine rechte Seitenkante (20) aufweist, welche teilweise eine jeweilige linke und rechte Beinöffnung (22) des Hauptkörpers (12) definieren;
ein saugfähiges Material (26), welches zumindest am Zwickel (18) mit einer Innenseite (13) des Hauptkörpers (12) gekoppelt ist;
ein wasserdichtes Material (28), welches zwischen dem Hauptkörper (12) und dem saugfähigen Material (26) eingeschoben ist; und
ein linkes und ein rechtes Gummiband (30), welche mit äußeren Seitenkanten des saugfähigen Materials (26) gekoppelt sind;
wobei das linke und rechte Gummiband (30) an das wasserdichte Material (28) verklebt sind; und
wobei das wasserdichte Material (28) an dem Hauptkörper (12) in der Nähe der jeweiligen linken und rechten Seitenkante (20) des Zwickels (18) befestigt ist;
**dadurch gekennzeichnet, dass**
das linke und rechte Gummiband (30) um jeweilige linke und rechte Seitenkanten des saugfähigen Materials (26) gefaltet sind; und
das wasserdichte Material (28) an dem Hauptkörper (12) zwischen jeweiligen unteren Hälften der Falten jedes des linken und rechten Gummibands (30) und der Innenseite (13) des Hauptkörpers (12) befestigt ist.

2. Kleidungsstück nach Anspruch 1, wobei das wasserdichte Material (28) unter Verwendung einer Klebefolie (36) an seitlichen äußeren Kanten des wasserdichten Materials (28) jeweils mit dem linken und rechten Gummiband (30) verklebt ist, und sich das wasserdichte Material (28) über die Klebefolie (36) nach außen hinaus erstreckt, um freie Kanten bereitzustellen, welche an dem Hauptkörper (12) befestigt sind.

3. Kleidungsstück nach Anspruch 1, wobei das linke und rechte Gummiband (30) an das saugfähige Material (26) verklebt ist, wobei das linke und rechte Gummiband (30) die jeweiligen linke und rechte Seitenkante des saugfähigen Materials (26) überlappen.

4. Kleidungsstück nach einem der vorstehenden Ansprüche, weiter ein Futtermaterial (24) umfassend, welches mit dem saugfähigen Material (26) gekoppelt ist, sodass das saugfähige Material (26) zwischen dem Futtermaterial (24) und dem wasserdichten Material (28) eingeschoben ist.

5. Kleidungsstück nach Anspruch 4, wobei das linke und rechte Gummiband (30) um die jeweiligen linken und rechten Seitenkanten jedes des Futtermaterials (24) und des saugfähigen Materials (26) gefaltet sind; und
wobei das linke und rechte Gummiband (30) an das Futtermaterial (24) und das saugfähige Material (26) verklebt sind, wobei das linke und rechte Gummiband (30) die jeweiligen linken und rechten Seitenkanten jedes des Futtermaterials (24) und des saugfähigen Materials (26) überlappen.

6. Kleidungsstück nach einem der vorstehenden Ansprüche, wobei linke und rechte Seitenkanten (48) eines Materials des Hauptkörpers (12) am Zwickel (18) zu der Innenseite (13) des Hauptkörpers (12) hin umgeschlagen sind, um die linke und rechte Seitenkante (20) des Zwickels (18) zu definieren; und
wobei linke und rechte Seitenkanten des wasserdichten Materials (28) an der jeweiligen linken und rechten Seitenkante (48) des Materials des Hauptkörpers (12) befestigt sind.

7. Kleidungsstück nach Anspruch 6, wobei die linke und rechte Seitenkante des wasserdichten Materials (28) durch Nähen (39) an der jeweiligen linken und rechten Seitenkante (48) des Materials des Hauptkörpers (12) befestigt sind.

8. Kleidungsstück nach Anspruch 7,
wobei das linke und rechte Gummiband (30) jeweils die Nähte (39) zwischen der linken und rechten Seitenkante des wasserdichten Materials (28) und der jeweiligen linken und rechten Seitenkante (48) des Materials des Hauptkörpers (12) überlappen.

9. Kleidungsstück nach einem der Ansprüche 6 bis 8, wobei das Material des Hauptkörpers (12) dort mit sich selbst verklebt ist, wo die linke und rechte Seitenkante (48) des Materials des Hauptkörpers (12) umgeschlagen sind, und der Hauptkörper (12) auf einer Außenseite (15) davon zumindest am Zwickel (18) im Wesentlichen nahtlos ist.

10. Verfahren zur Herstellung eines saugfähigen Kleidungsstücks (10) nach einem der obigen Ansprüche, wobei das Verfahren umfasst:
Bereitstellen des Hauptkörpers (12);
Bereitstellen des saugfähigen Materials (26), welches so bemessen und geformt ist, dass es auf den Zwickel (18) passt und eine linke und eine rechte Seitenkante aufweist, welche so konfiguriert sind, dass sie sich entlang der jeweiligen linken und rechten Seitenkante (20) des Zwickels (18) erstrecken;
Befestigen des linken und rechten Gummibands (30) entlang der jeweiligen linken und rechten Seitenkante des saugfähigen Materials (26).
Bereitstellen des wasserdichten Materials (28), welches so bemessen und geformt ist, dass es auf den Zwickel (18) passt und eine linke und eine rechte Seitenkante aufweist, welche so konfiguriert sind, dass sie sich entlang der jeweiligen linken und rechten Seitenkante (20) des Zwickels (18) erstrecken;
Befestigen der linken und rechten Seitenkante des wasserdichten Materials (28) an dem Hauptkörper (12) in der Nähe der jeweiligen linken und rechten Seitenkante (20) des Zwickels (18), sodass sich das wasserdichte Material (28) an der Innenseite (13) des Hauptkörpers (12) am Zwickel (18) befindet; und
Platzieren des befestigten saugfähigen Materials (26) und des linken und rechten Gummibands (30) auf dem Zwickel (18) über dem wasserdichten Material (28) und Befestigen des linken und rechten Gummibands (30) an dem wasserdichten Material (28), sodass das wasserdichte Material (28) zwischen dem saugfähigen Material (26) und dem Hauptkörper (12) eingeschoben ist.

11. Verfahren nach Anspruch 10, weiter Umschlagen linker und rechter Seitenkanten (48) eines Materials des Hauptkörpers (12) am Zwickel (18) zu der Innenseite (13) des Hauptkörpers (12) hin umfassend, um die linke und rechte Seitenkante (20) des Zwickels (18) zu definieren; und
Befestigen der linken und rechten Seitenkante des wasserdichten Materials (28) an der jeweiligen linken und rechten Seitenkante (48) des Materials des Hauptkörpers (12).

12. Verfahren nach Anspruch 11, wobei das Befestigen der linken und rechten Seitenkante des wasserdichten Materials (28) an der jeweiligen linken und rechten Seitenkante (48) des Materials des Hauptkörpers (12) durch Nähen erfolgt.

13. Verfahren nach Anspruch 12, wobei das linke und rechte Gummiband (30) jeweils die Nähte (39) zwischen der linken und rechten Seitenkante des wasserdichten Materials (28) und der jeweiligen linken und rechten Seitenkante (48) des Materials des Hauptkörpers (12) überlappen.

14. Verfahren nach einem der Ansprüche 11 bis 13, weiter Verkleben des Materials des Hauptkörpers (12) dort mit sich selbst umfassend, wo die linke und rechte Seitenkante (48) des Materials des Hauptkörpers (12) umgeschlagen sind, sodass der Hauptkörper (12) auf einer Außenseite (15) davon zumindest am Zwickel (18) im Wesentlichen nahtlos ist.

15. Verfahren nach einem der Ansprüche 10 bis 14, weiter umfassend:
Bereitstellen eines Futtermaterials (24), welches so bemessen und geformt ist, dass es auf den Zwickel (18) passt und eine linke und eine rechte Seitenkante aufweist, welche so konfiguriert sind, dass sie sich entlang der jeweiligen linken und rechten Seitenkante (20) des Zwickels (18) erstrecken;
Befestigen des linken und rechten Gummibands (30) entlang der jeweiligen linken und rechten Seitenkanten jedes des saugfähigen Materials (26) und des Futtermaterials (24).
Platzieren des befestigten Futtermaterials (24), saugfähigen Materials (26) und linken und rechten Gummibands (30) auf dem Zwickel (18) über dem wasserdichten Material (28) und Befestigen des linken und rechten Gummibands (30) an dem wasserdichten Material (28), sodass das saugfähige Material (26) zwischen dem Futtermaterial (24) und dem wasserdichten Material (28) eingeschoben ist.

## Revendications

1. Vêtement (10), comprenant :
un corps principal (12) incluant une partie avant (14), une partie arrière (16) et un soufflet (18) reliant la partie avant (14) et la partie arrière (16), dans lequel le soufflet (18) comporte des bords latéraux gauche et droit (20) définissant partiellement des ouvertures de jambe gauche et droite respectives (22) du corps principal (12) ;
un matériau absorbant (26) couplé à une face interne (13) du corps principal (12) au moins au niveau du soufflet (18) ;
un matériau imperméable (28) pris en sandwich entre le corps principal (12) et le matériau absorbant (26) ; et
des bandes élastiques gauche et droite (30) couplées aux bords latéraux externes du matériau absorbant (26) ;
dans lequel les bandes élastiques gauche et droite (30) sont liées au matériau imperméable (28) ; et
dans lequel le matériau imperméable (28) est fixé au corps principal (12) à proximité des bords latéraux gauche et droit respectifs (20) du soufflet (18) ;
**caractérisé en ce que**
les bandes élastiques gauche et droite (30) sont repliées autour des bords latéraux gauche et droit respectifs du matériau absorbant (26) ; et
le matériau imperméable (28) est fixé au corps principal (12) entre des moitiés inférieures respectives des plis de chacune des bandes élastiques gauche et droite (30) et la face interne (13) du corps principal (12).

2. Vêtement selon la revendication 1, dans lequel le matériau imperméable (28) est lié à chacune des bandes élastiques gauche et droite (30) au niveau des bords latéraux externes du matériau imperméable (28) à l'aide d'un film de liaison (36) et le matériau imperméable (28) s'étend vers l'extérieur au-delà du film de liaison (36) pour fournir des bords libres qui sont fixés au corps principal (12).

3. Vêtement selon la revendication 1, dans lequel les bandes élastiques gauche et droite (30) sont liées au matériau absorbant (26) où les bandes élastiques gauche et droite (30) chevauchent les bords latéraux gauche et droit respectifs du matériau absorbant (26).

4. Vêtement selon l'une quelconque des revendications précédentes, comprenant en outre un matériau de doublure (24) couplé au matériau absorbant (26) de telle sorte que le matériau absorbant (26) soit pris en sandwich entre le matériau de doublure (24) et le matériau imperméable (28).

5. Vêtement selon la revendication 4, dans lequel les bandes élastiques gauche et droite (30) sont repliées autour des bords latéraux gauche et droit respectifs de chacun du matériau de doublure (24) et du matériau absorbant (26) ; et
dans lequel les bandes élastiques gauche et droite (30) sont liées au matériau de doublure (24) et au matériau absorbant (26) où les bandes élastiques gauche et droite (30) chevauchent les bords latéraux gauche et droit respectifs de chacun du matériau de doublure (24) et du matériau absorbant (26).

6. Vêtement selon l'une quelconque des revendications précédentes, dans lequel les bords latéraux gauche et droit (48) d'un matériau du corps principal (12) sont repliés vers la face interne (13) du corps principal (12) au niveau du soufflet (18) pour définir les bords latéraux gauche et droit (20) du soufflet (18) ; et
dans lequel les bords latéraux gauche et droit du matériau imperméable (28) sont fixés aux bords latéraux gauche et droit respectifs (48) du matériau du corps principal (12).

7. Vêtement selon la revendication 6, dans lequel les bords latéraux gauche et droit du matériau imperméable (28) sont fixés aux bords latéraux gauche et droit respectifs (48) du matériau du corps principal (12) par une couture (39).

8. Vêtement selon la revendication 7,
dans lequel les bandes élastiques gauche et droite (30) chevauchent respectivement la couture (39) entre les bords latéraux gauche et droit du matériau imperméable (28) et les bords latéraux gauche et droit respectifs (48) du matériau du corps principal (12).

9. Vêtement selon l'une quelconque des revendications 6 à 8, dans lequel le matériau du corps principal (12) est lié à lui-même où les bords latéraux gauche et droit (48) du matériau du corps principal (12) sont repliés, et le corps principal (12) est sensiblement sans couture sur une face externe (15) de celui-ci au moins au niveau du soufflet (18).

10. Procédé de fabrication d'un vêtement absorbant (10) selon l'une quelconque des revendications précédentes, le procédé comprenant :
la fourniture du corps principal (12) ;
la fourniture du matériau absorbant (26), qui est dimensionné et façonné pour s'adapter au soufflet (18) et qui comporte des bords latéraux gauche et droit configurés pour s'étendre le long des bords latéraux gauche et droit respectifs (20) du soufflet (18) ;
la fixation des bandes élastiques gauche et droite (30) le long des bords latéraux gauche et droit respectifs du matériau absorbant (26) ;
la fourniture du matériau imperméable (28), qui est dimensionné et façonné pour s'adapter au soufflet (18) et qui comporte des bords latéraux gauche et droit configurés pour s'étendre le long des bords latéraux gauche et droit respectifs (20) du soufflet (18) ;
la fixation des bords latéraux gauche et droit du matériau imperméable (28) au corps principal (12) à proximité des bords latéraux gauche et droit respectifs (20) du soufflet (18) de telle sorte que le matériau imperméable (28) soit situé sur la face interne (13) du corps principal (12) au niveau du soufflet (18) ; et
le placement du matériau absorbant (26) et des bandes élastiques gauche et droite (30) sur le soufflet (18) au-dessus du matériau imperméable (28) et la fixation des bandes élastiques gauche et droite (30) au matériau imperméable (28) de telle sorte que le matériau imperméable (28) soit pris en sandwich entre le matériau absorbant (26) et le corps principal (12).

11. Procédé selon la revendication 10, comprenant en outre le pliage des bords latéraux gauche et droit (48) d'un matériau du corps principal (12) vers la face interne (13) du corps principal (12) au niveau du soufflet (18) pour définir les bords latéraux gauche et droit (20) du soufflet (18) ; et
la fixation des bords latéraux gauche et droit du matériau imperméable (28) aux bords latéraux gauche et droit respectifs (48) du matériau du corps principal (12).

12. Procédé selon la revendication 11, dans lequel la fixation des bords latéraux gauche et droit du matériau imperméable (28) aux bords latéraux gauche et droit respectifs (48) du matériau du corps principal (12) est réalisée par une couture.

13. Procédé selon la revendication 12, dans lequel les bandes élastiques gauche et droite (30) chevauchent respectivement la couture (39) entre les bords latéraux gauche et droit du matériau imperméable (28) et les bords latéraux gauche et droit respectifs (48) du matériau du corps principal (12).

14. Procédé selon l'une quelconque des revendications 11 à 13, comprenant en outre la liaison du matériau du corps principal (12) à lui-même où les bords latéraux gauche et droit (48) du matériau du corps principal (12) sont repliés de telle sorte que le corps principal (12) soit sensiblement sans couture sur une face externe (15) de celui-ci au moins au niveau du soufflet (18).

15. Procédé selon l'une quelconque des revendications 10 à 14, comprenant en outre :
la fourniture d'un matériau de doublure (24) dimensionné et façonné pour s'adapter au soufflet (18) et comportant des bords latéraux gauche et droit configurés pour s'étendre le long des bords latéraux gauche et droit respectifs (20) du soufflet (18) ;
la fixation des bandes élastiques gauche et droite (30) le long des bords latéraux gauche et droit respectifs de chacun du matériau absorbant (26) et du matériau de doublure (24) ; et
le placement du matériau de doublure (24), du matériau absorbant (26) et des bandes élastiques gauche et droite (30) sur le soufflet (18) au-dessus du matériau imperméable (28) et la fixation des bandes élastiques gauche et droite (30) au matériau imperméable (28) de telle sorte que le matériau absorbant (26) soit pris en sandwich entre le matériau de doublure (24) et le matériau imperméable (28).
